# EUROPEAN PATENT APPLICATION

(11) **EP 1 715 034 A1**
(43) Date of publication of application: **25.10.2006**
(21) Application number: 05709992.1
(22) Date of filing: 09.02.2005
(51) Int. Cl.: C12N 5/08, C12Q 1/02, A61K 35/12, A61K 45/00, A61P 7/00, A61P 37/06

(54) **REGULATORY CELL CONTROLLING IMMUNOLOGICAL ACTIVITY OF T CELL**

(30) Priority: 12.02.2004 JP 2004034367
(71) Applicant: ImmunoFrontier, Inc., Tsu-shi, Mie 514-0061 (JP); Suzuki, Haruhiko, Nagoya-shi, Aichi 465-0048 (JP)
(72) Inventor: SUZUKI, Haruhiko, 4650048 (JP)
(74) Representative: Steinecke, Peter
(86) International application number: PCT/JP2005/001940
(87) International publication number: WO 2005/078072

(57) **Abstract**

[Subject] To provide regulatory T cells that suppress activated CD8⁺ killer T cells with tissue-damaging or cytotoxic effects. [Solution means] CD8⁺CD122⁺ T cell subsets are provided as regulatory T cells that suppress activity of activated CD8⁺ killer T cells. Administration of these T cell subsets can suppress tissue/cell damages. In addition, it has become possible to explore agents that augment immunosuppressive activity of these T cell subsets by using the experimental method described in the present invention.

## Description

### [Field of invention]

The present invention relates to T cell subsets involved in the suppression of immune responses within the living body and their application to treatment of disease.

### [Background art]

These immune responses are induced and regulated by interactions among B lymphocytes, T lymphocytes, antibodies, and antigen-presenting cells (APC). First, foreign antigens undergo processing by APC, and are bound with major histocompatibility complex (MHC) class II molecules to be presented to helper T cells. After the foreign antigens bound with MHC are recognized by helper T cells, T cell activation occurs. Cytokines excreted by activated T cells stimulate differentiation of killer T cells as well as promote the differentiation of antigenically-stimulated B cells into antibody-producing cells.
Cells expressing antigens are rejected by excreted antibodies and activated killer T cells, and cellular and humoral responses to reject foreign antigens proceed. In other words, T cells play a central role in recognizing target antigens and inducing immune responses. For example, CD4⁺ T cells and CD8⁺ T cells have traditionally been known to play a critical role also in antitumor immune responses.

CD8⁺ CTLs (cytotoxic T cells) are key effector cells with an ability to directly destroy tumor cells both in vivo and in vitro. These cells have high specificity to antigen peptides presented by MHC class I. In contrast, natural killer T (NKT) cells have low antigen specificity, and are considered to be effector cells that exhibit particular immune responses (refer to nonpatent literature 1). Meanwhile, CD4⁺ T cells do not directly destroy tumor cells, but they are assumed to play a fundamental role via multiple mechanisms to control antitumor immune responses (refer to nonpatent literature 2). CD4⁺ helper T cells that recognize tumor antigen peptides presented by MHC class II molecules augment the activation and growth of killer T cells via interactions with antigen-presenting cells (APC).

It has been shown that CD4⁺CD25⁺ regulatory T cells (Treg) are effective in suppressing the progression of antitumor immune responses and various autoimmune diseases (refer to patent literature 1 and nonpatent literature 3). However, CD4⁺CD25⁺ T cells suppress cytotoxic CD8⁺ killer T cells not by directly acting on them, but via targeting CD4⁺ helper T cells to suppress their helper functions. Therefore it is considered impossible for CD4⁺CD25⁺ cells to suppress already-activated CD8⁺ killer T cells.

It is commonly known that various T cells, NK cells, NK T cells, and dendritic cells, besides CD4⁺CD25⁺ T cells, have regulatory functions on immune responses. Among these various regulatory cells, CD8⁺ suppressor T cells, in particular, have been considered to have suppressive functions on immune responses. Many studies on these cells have been conducted for long years. However, these could not be isolated and specifically identified, and were forgotten. In this regard, many reports on Vα14+ NKT cell population, discovered in mice by Taniguchi et al., have been published in association with the development of autoimmune diseases, while similar NK T cells have been reported to be specifically reduced in the condition of autoimmune disease in human. It has also been demonstrated that activated Vα14+ NKT cells are involved in surviving of engrafted tissue and inhibition of IgE production, and at the same time, have potent cytotoxic activity and cause fulminant hepatitis. Activated Vα14+ NKT cells are considered to secrete cytokines such as interferon-γ and IL-4, and regulate the immune system by balancing them, but details of its molecular mechanisms remain elucidated.

[Patent literature 1] Patent application US2003049696
[Patent literature 2] Patent application US6531453
[Nonpatent literature 1] M. J. Smythet al. , J. Exp. Med. 191(2000), pp661-668
[Nonpatent literature 2] R. F. Wang, Trends. Immunol. 5(2001), pp269-276
[Nonpatent literature 3] S. Sakaguchi et al, Immunol. Rev. 182(2001), pp18-32
[Nonpatent literature 4] M. Taniguchi et al, Annu. Rev. Immunol. 21(2001), pp83-513

### [Disclosure of the Invention]

### [Problems to be Solved by the Invention]

CD8⁺ killer T cells play a central role in damaging tissue and cells in autoimmune diseases and transplantation rejection reactions. A method to specifically suppress activated CD8⁺ killer T cells remains undiscovered. CD4⁺CD25⁺ T cells have preventive effects to suppress the activation of CD8⁺ killer T cells indirectly via regulation of CD4⁺ helper T cells. However, CD4⁺CD25⁺ T cells are considered to be not necessarily effective in suppressing already activated CD8⁺ killer T cells. Inhibitory NK T cells such as mouse Vα14+ NKT cells regulate immunity via balancing of cytokine secretion, therefore they are not supposed to directly and specifically control CD8⁺ killer T cells either, and various responses are predicted.

### [Means of solving the problems]

The inventors have focused attention to mice with various abnormalities in the hemopoietic cells, abnormally activated and increased T cells in the lymph node, and severe anemia due to autoimmune hemolysis, caused by the lack of CD122 (IL-2/IL-15 receptor β chain). The results of keen examination on recovery from the above abnormal phenotypes of these mice revealed that normal mice could be obtained by administering CD8⁺CD122⁺ T cells isolated from normal mice with intact CD122 into newborn mice lacking CD122, which lead to the present invention.

In addition, the inventors demonstrated the immunosuppressive activity of CD8⁺CD122⁺ T cells in vitro by measuring interferon-γ produced within CD8⁺ killer T cells that coexisted with CD8⁺CD122⁺ T cells under culture conditions where isolated CD8⁺killer T cells were activated in vitro. Also, when CD4⁺ helper T cells were used instead of CD8⁺ killer T cells, immunosuppressive activity of CD8⁺CD122⁺ T cells was similarly demonstrated by measuring interleukin-2 produced by CD4⁺ helper T cells. Also, suppression of the cytotoxic activity of NK cells was demonstrated. These CD8⁺CD122⁺ T cells with immunosuppressive activity themselves can be used as immunosuppressive agents. In addition, agents that activate or enhance CD8⁺CD122⁺ T cells can be explored using this immunosuppressive activity as a marker.

As described above, the present invention provides (1) immunosuppressive agents containing T cell subsets with CD8⁺CD122⁺ surface markers, and (2) immunosuppressive agents consisting of agents that activate the said subsets. By administering the said immunosuppressive agents to individuals, activity of CD8⁺ killer T cells can be suppressed, which results in specific suppression of immune responses that damaged the individuals. Therefore, the present invention provides a method to suppress immune responses in mammals, including administration of the above immunosuppressive agents to mammals. In addition, the present invention provides methods, including administering pharmacologically effective doses of the said immunosuppressive agents to mammals, to treat or prevent immunologic abnormalities such as autoimmune diseases, transplantation rejection reactions, graft-versus-host reactions, and hematopoietic injuries. In addition, the present invention provides applications of the said immunosuppressive agents to therapeutic or preventive agents against immunologic abnormalities such as autoimmune diseases, transplantation rejection reactions, graft-versus-host reactions, and hematopoietic injuries.

### [Effects of the Invention]

An imbalance in CD8⁺ cell subset caused by predominant increase of activated CD8⁺CD122⁻ killer T cell subsets in comparison with CD8⁺CD122⁺ T cells, results in abnormal immune responses. It was demonstrated that administering CD8⁺CD122⁺ T cells improved these abnormalities. Therefore, the effects of the present invention are to compensate the lack of quantity or activity of CD8⁺CD122⁺ T cells, which causes the immunologic abnormalities that result from excessive activation of CD8⁺CD122⁻ killer T cells for some reasons, including various autoimmune diseases, transplantation rejection reactions, graft-versus-host reactions, and hematopoietic injuries, and to treat or prevent the said immunologic abnormalities.

### [Brief description of the drawings]

[Fig. 1] Fig. 1 shows lethal effects on mice from CD8⁺CD122⁻ T cells.
[Fig. 2] Fig. 2 shows the effects of CD8⁺CD122⁺ T cells on suppressing interferon-γ production from CD8⁺ cells and CD4⁺ T cells.
[Fig. 3] Fig. 3 shows the effects of CD8⁺CD122⁺ T cells on normalizing T cells of CD122 knockout mice.
[Fig. 4] Fig. 4 shows the effects of CD8⁺CD122⁺ T cells on normalizing granulocytes of CD122 knockout mice.
[Fig. 5] Fig. 5 shows the effects of CD8⁺CD122⁺ T cells on normalizing erythrocytes of CD122 knockout mice.
[Fig. 6] Fig. 6 shows the effects of CD8⁺CD122⁺ T cells on suppressing cytokine production of particular antigen-specific T cells.
[Fig. 7] Fig. 7 shows the effect of CD8⁺CD122⁺ T cells on suppressing cell proliferation.
[Fig. 8] Fig. 8 shows suppression of induction of cytotoxic T lymphocytes (CTLs) by CD8⁺CD122⁺ T cells.
[Fig. 9] Fig. 9 shows suppression of NK cells by CD8⁺CD122⁺ T cells.

### [Best Mode for Carrying Out the Invention]

Immunosuppressive agents in the present invention can be prepared by isolating CD8⁺CD122⁺ T cells from the recipient individuals. For example, in cases of individuals who are scheduled to undergo organ or tissue transplantation, autologous blood is collected before conducting transplantation, and lymphocyte fractions are obtained by density-gradient centrifugation using the differences in specific gravity of autologous blood, subsequently, CD8⁺CD122⁺ T cell fractions are obtained aseptically using magnetic bead-bound antibodies and a cell sorter. The CD8⁺CD122⁺ T cell fractions obtained are suspended in an appropriate culture medium containing cytokines such as interleukin-2 and are cultured to grow. Transplantation rejection reactions and graft-versus-host reactions can be suppressed by administering the amplified cells into the individuals during or after the transplantation.

Also, administering the said isolated cells into immunodeficient mice such as NOG mice can be used as a method to grow CD8⁺CD122⁺ T cells. In the pilot tests using NOG mice, ≥ 10 fold increase of human CD8⁺ T cells, administered into these mice, was confirmed after 7-8 weeks. Human CD8⁺ T cells can be isolated from the mouse spleen by cell sorting using antihuman CD8 antibodies.

Also, when autoimmune diseases develop unexpectedly, damaging reactions to self-tissues can be sedated by returning CD8⁺CD122⁺ T cells into the diseased individual as immunosuppressive agents, which are isolated from autologous blood collected from the said individual and grown as described above.

Also, immunosuppressive agents of the present invention can be screened and selected by measuring the reduction level of interferon-γ production from CD8⁺CD122⁻T cells that were isolated from an individual and cultured with coexisting CD8⁺CD122⁺ T cells under stimulatory conditions with anti-CD3 antibody or cytokines such as interleukin-2, compared with those under nonexistence of CD8⁺CD122⁺ T cells, or by measuring the suppressive activity of CD8⁺CD122⁺ T cells that were pretreated with candidate substances. When CD4⁺CD25⁻ T cells are used instead of CD8⁺CD122⁻ T cells, immunosuppressive agents can similarly be screened and selected by using reduced interleukin-2 production as a marker. In addition, immunosuppressive agents can be screened and selected with NK cells by using their cytotoxic activity as a marker.

### [Example 1]

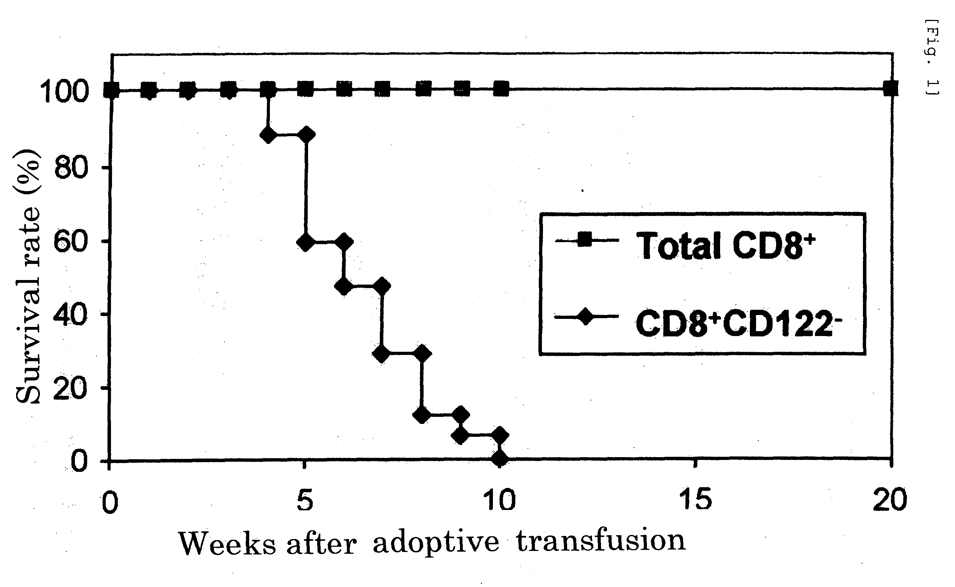

Half a million CD8⁺CD122⁻ T cells or total CD8⁺ T cells isolated from normal mice by using a cell sorter were intravenously transfused into lymphocyte-lacking RAG-2 knockout mice, and survival rates of the mice were followed for 20 weeks after transfusion. As shown in the results of Fig. 1, all the mice transfused with CD8⁺CD122⁻ T cells (17 mice) died within 10 weeks after transfusion, while all the mice transfused with total CD8⁺ T cells (20 mice) were healthy until 20 weeks after transfusion.

### [Example 2]

50,000 CD8⁺CD122⁻ T cells isolated from normal mice by using a cell sorter were stimulated by anti-mouse CD3 antibodies immobilized onto a culture plate, and cultured in the presence of interleukin-2 (25 U/mL) for 3 days. Cells were collected after the culture, and fixed after staining the cell surface with anti-CD8 antibodies, and flow cytometric analysis was performed on cells that were stained for intracellular interferon-γ with anti-interferon-γ antibodies. The same experiment was performed by adding 10,000 CD8⁺CD122⁺ T cells, and interferon-γ (IFN-γ) production from CD8⁺CD122⁻ T cells was examined.

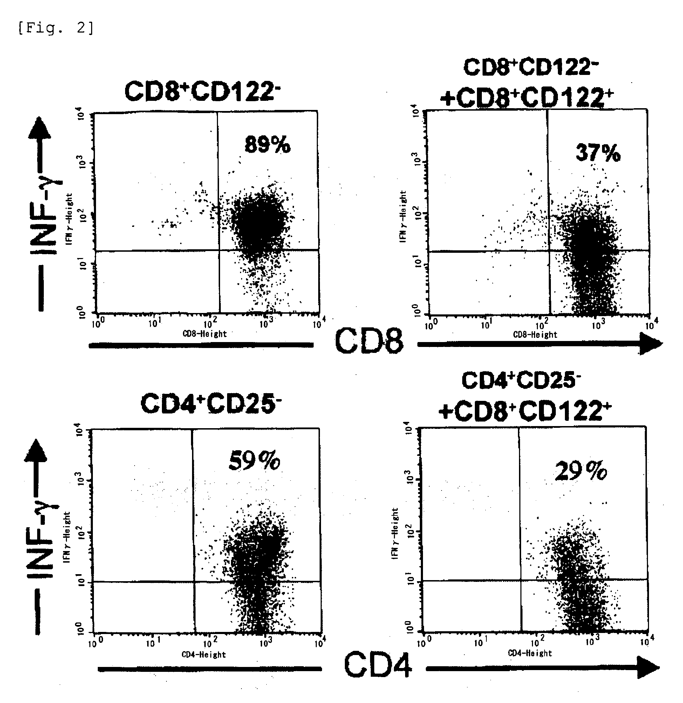

The results are shown in Fig. 2 (The values in the panel of the figure show the percentages of interferon-γ-producing cells). Comparison of the results after both cultures showed a lower percentage of interferon-γ-producing cells by the effects of added CD8⁺CD122⁺ T cells. In addition, when the effects of CD8⁺CD122⁺ T cells were similarly investigated by using CD4⁺CD25⁻ T cells, instead of CD8⁺CD122⁻ T cells, it was revealed that CD8⁺CD122⁺ T cells also had inhibitory effects on interferon-γ production from CD4⁺ T cells.

### [Example 3]

50,000 cells isolated from normal mice by using a cell sorter (CD8⁺CD122⁺, CD8⁺CD122⁻, and CD4⁺CD25⁺ cells) were subcutaneously injected into neonatal CD122 knockout mice. After 7 weeks, CD4⁺ T cells of the knockout mice spleen were stained with anti-CD69 antibody, and flow cytometric analysis was performed to examine the activated state of the T cells. At the same time, peripheral blood granulocyte count and hematocrit were measured.

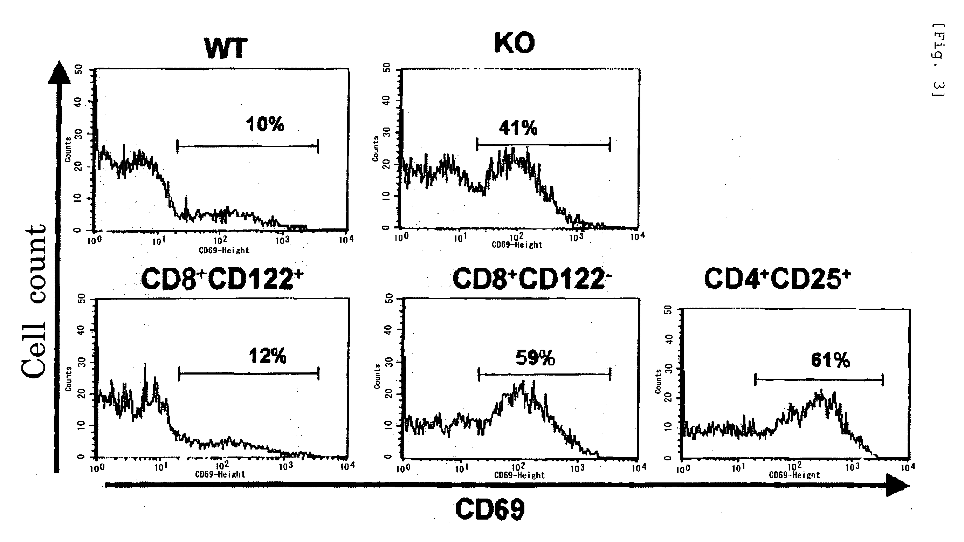

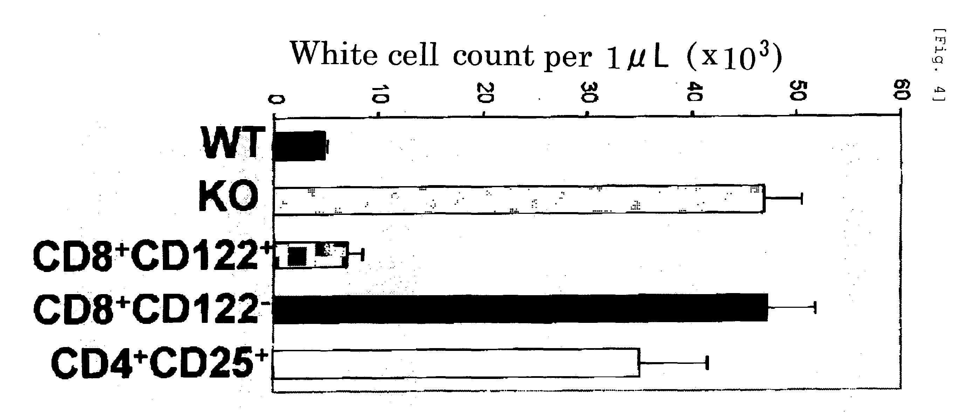

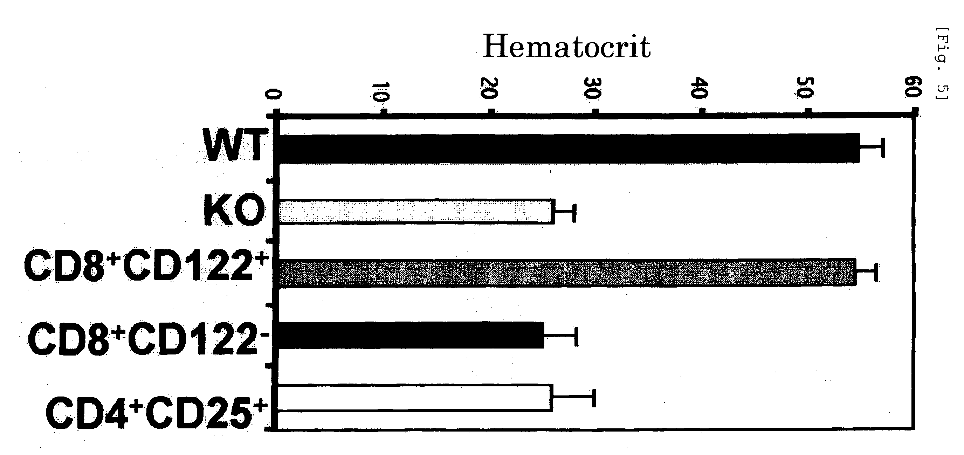

The results are shown in Figs. 3-5. Here, the values in the panel of Fig. 3 present the percentages of activated CD69⁺ T cells. The upper two panels present cases of untreated normal mice (WT) and CD122 knockout mice (KO). The lower panel presents conditions of knockout mice to which each cell was transfused in the neonatal period. Fig. 4 presents mean values of peripheral blood granulocyte counts in 5 cases of untreated normal mice (WT), CD122 knockout mice, and knockout mice transfused with each T cell subset. In addition, Fig. 5 presents mean values of hematocrit readings in 5 cases of untreated normal mice (WT), CD122 knockout mice, and knockout mice transfused with each T cell subset.

These results show only CD8⁺CD122⁺ cell transfusion corrected the T cell activity of knockout mice to close to normal. In addition, it was demonstrated that increased granulocytes and anemia observed in untreated knockout mice could be corrected by CD8⁺CD122⁺ cell transfusion.

### [Example 4]

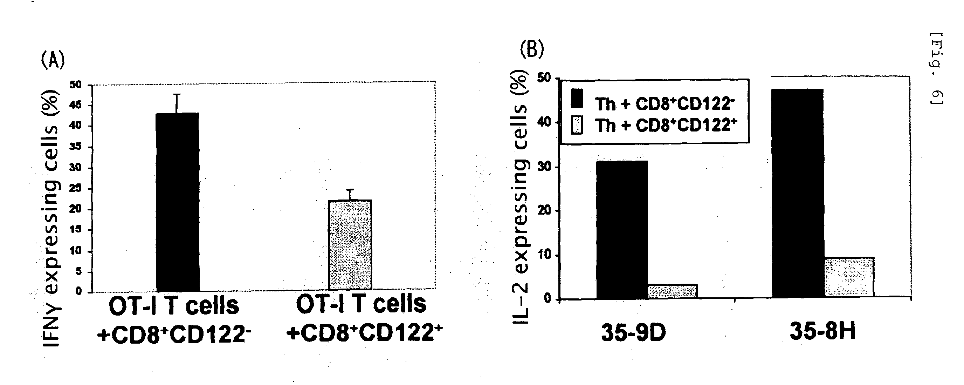

T cells of transgenic mice (OT-1) that were produced with T cell receptors that specifically react with constitutive peptides of egg albumin (OVA) were cultured under stimulation by OVA peptides. After CD8⁺CD122⁺ cells or CD8⁺CD122⁻ cells collected from wild type B6 mice were added to this culture and cocultured for 48 hours, IFN γ production from transgenic mouse T cells was analyzed by intracellular cytokine staining and FACS, and the percentage of IFN γ-producing cells was calculated. The results are shown in Fig. 6 (A). They showed that the percentage of IFN γ-producing cells significantly reduced in those cocultured with CD8⁺CD122⁺ cells. Next, 2 types of OVA peptide-specif ic helper T cell clones (35-9D and 35-8H) were cocultured with CD8⁺CD122⁺ cells or CD8⁺CD122⁻ cells of wild type mice under stimulation by OVA peptides. The measurement results of IL-2 production from helper T cell clones after the culture are shown in Fig. 6 (B). The percentage of IL-2 producing cells was significantly reduced in the clone cocultured with CD8⁺CD122⁺ cells.

### [Example 5]

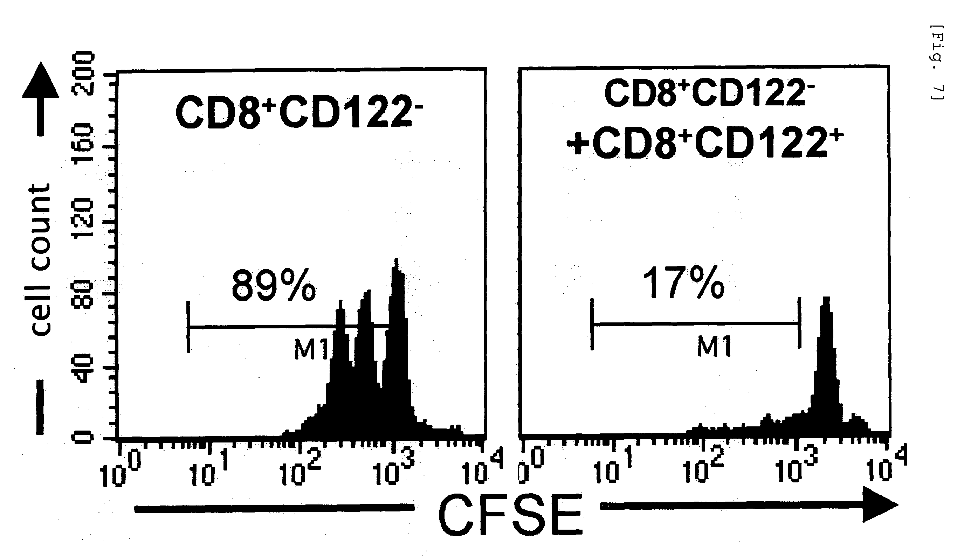

CD8⁺CD122⁻ cells collected from B6 mice were CFSE-fluorescence labeled, and cultured under stimulation by immobilized anti-CD3 antibodies for 48 hours. The results are shown in Fig. 7. In the single culture of CD8⁺CD122⁻ cells, proliferating or dividing cells with reduced CFSE fluorescence were noted. Meanwhile, no reduced CFSE fluorescence was noted in CD8⁺CD122⁻ cells cocultured with CD8⁺CD122⁺ cells (1/4 of CD8⁺CD122⁻ cells), demonstrating that no cell proliferation occurred.

### [Example 6]

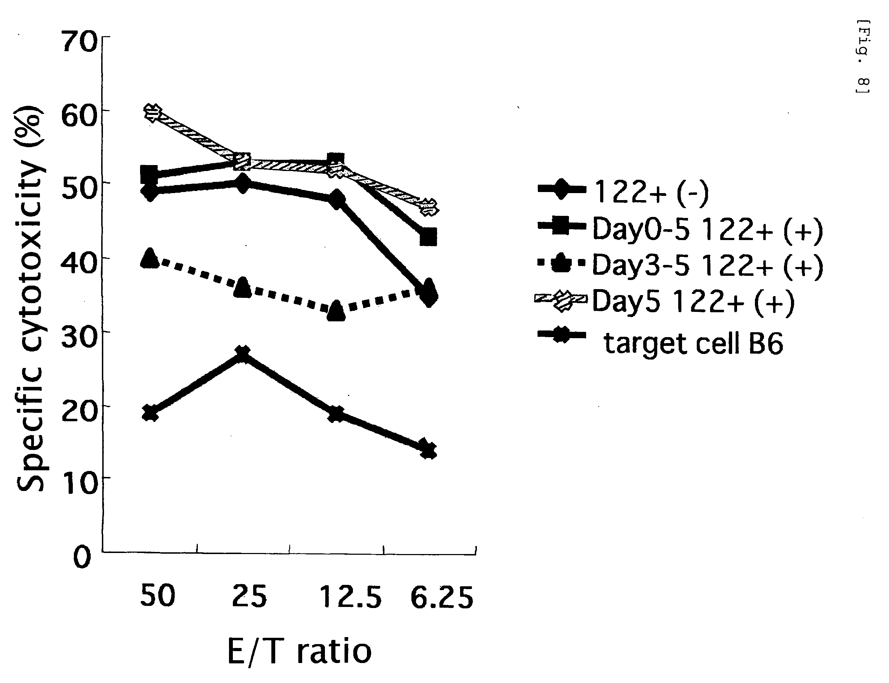

Mixed lymphocyte culture (MLC) of T cells collected from B6 mice was performed with irradiated BALB/c mouse spleen cells for 5 days, and allo-specific CTLs were induced. After CD8⁺CD122⁺ cells collected from B6 mice were added on day 0-5, day 3-5, or day 5 after starting the MLC, cytotoxicity tests targeting blasted BALB/c cells were performed. The results are shown in Fig. 8. The group to which CD8⁺CD122⁺ cells were added on MLC day 3 showed significantly reduced CTL activity, compared with the nonadded group.

### [Example 7]

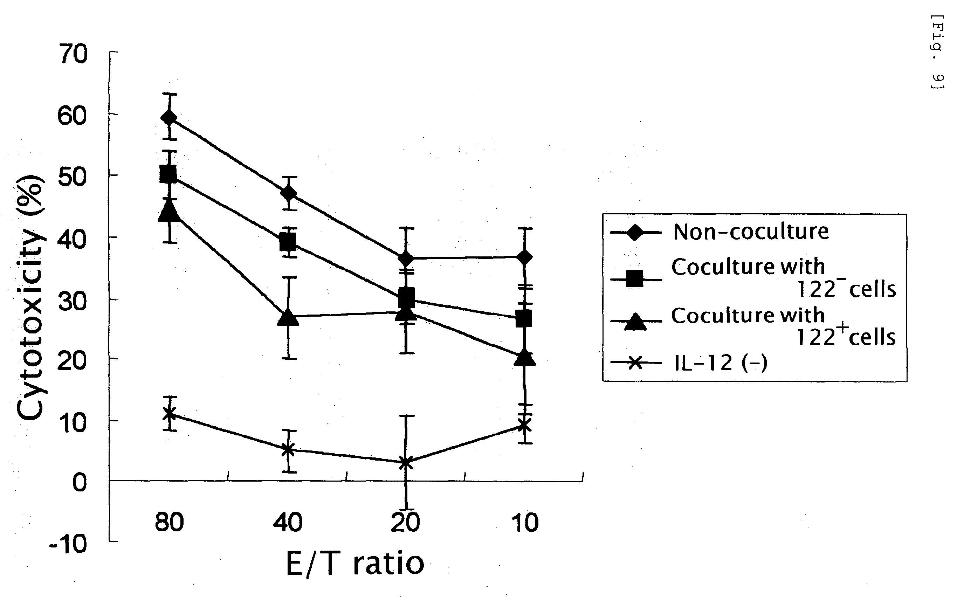

Spleen cells were collected after intraperitoneal administration of poly [I] : [C] into B6 mice, and cultured in the presence of IL-12 for 42 hours to induce activated NK cells. CD8⁺CD122⁺ cells or CD8⁺CD122⁻ cells prepared from B6 mice were added during the culture, and NK activity was measured after the culture by using YAC-1 as target cells. The results are shown in Fig. 9. It was demonstrated that induction of NK cell activity was more profoundly suppressed in the coculture with CD8⁺CD122⁺ cells than with CD8⁺CD122⁻ cells.

### [Industrial applicability]

The present invention can be applied to the treatment or prevention of severe immunologic disorders such as autoimmune diseases, transplantation rejection reactions, graft-versus-host reactions, and hematopoietic injury reaction.

## Claims

1. T cell subsets having a CD8⁺CD122⁺ cell surface marker, by which interferon-γ and/or interleukin-2 production activity of CD8⁺CD122⁻ T cells or CD4⁺CD25⁻ T cells can be suppressed.

2. Immunosuppressive agents that augment immunosuppressive activity of the T cell subsets of Claim 1.

3. Screening methods to select immunosuppressive agents of Claim 2 using immunosuppressive activity of the T cell subsets of Claim 1 as a marker.

4. Screening methods to select immunosuppressive agents of Claim 3 using interferon-γ and/or interleukin-2 production activity of CD8⁺CD122⁻ T cells or CD4⁺CD25⁻ T cells as a marker.

5. Treatment or prevention methods for autoimmune diseases wherein T cells with a CD8⁺CD122⁺ cell surface marker are administered to individuals with autoimmune disease, transplantation rejection reactions, graft-versus-host reactions, hematopoietic injuries, CD8⁺CD122⁻ T cells with excessively augmented or potentially augmented activity, or CD4⁺CD25⁻ T cells with excessively augmented or potentially augmented activity.

6. Treatment orpreventionmethods for autoimmune diseases of Claim 5, wherein CD8⁺CD122⁺ T cells that are isolated, or isolated and grown, from autologous peripheral blood are administered.
